# EUROPÄISCHE PATENTSCHRIFT

(11) **EP 2 010 923 B1**
(45) Veröffentlichungstag und Bekanntmachung des Hinweises auf die Patenterteilung: **11.06.2014**
(21) Anmeldenummer: 07724482.0
(22) Anmeldetag: 23.04.2007
(51) Int. Cl.: G01N 21/03, G01N 21/35

(54) **Lebensmittelanalysevorrichtung und Probentisch für diese**
Food analysing device and sample table for this device
Dispositif d'analyse de produit alimentaire et table d'échantillonage pour ce dispositif

(30) Priorität: 24.04.2006 DE 102006018926
(43) Veröffentlichungstag der Anmeldung: 07.01.2009
(73) Patentinhaber: Bruins, Hans Joachim, 81247 München (DE)
(72) Erfinder: Bruins, Hans Joachim, 81247 München (DE)
(74) Vertreter: Hertz, Oliver
(86) Internationale Anmeldenummer: PCT/EP2007/003552
(87) Internationale Veröffentlichungsnummer: WO 2007/121974

(56) Entgegenhaltungen:
- WO-A-03/029845
- WO-A-03/089912
- WO-A-2005/093433
- DE-A1- 3 628 155
- JP-A- 9 275 970
- US-A- 2 498 030
- US-A- 5 756 304
- US-B1- 7 105 338

## Beschreibung

Die Erfindung betrifft eine Lebensmittelanalysevorrichtung und einen Probentisch für diese.

Lebensmittel, wie beispielsweise Getreide, Getreideschrot, Hülsenfrüchte, Mehl, Ölsaaten, Fisch, sowie Fleisch und Fleischprodukte werden regelmäßig auf ihre Zusammensetzung hin analysiert. Dazu werden Spektrometer eingesetzt. Diese bestrahlen die zu untersuchende Probe des Lebensmittels mit einem Lichtstrahl sich ändernder Wellenlänge und messen die wellenlängenabhängige Reflexion beziehungsweise wellenlängenabhängige Absorption des Lichtstrahls durch die Probe des Lebensmittels.

Anhand des Reflexions- beziehungsweise Absorptionsprofils ist eine Aussage über die Zusammensetzung der Probe möglich.

Da Proben von Lebensmitteln in der Regel in ihren Eigenschaften inhomogen sind, ist es notwendig, entsprechende Messungen an einer Vielzahl von Positionen einer Probe durchzuführen, um so einen Mittelwert zu erhalten. Dazu wird der Lichtstrahl über die Probe geführt und an mehreren Stellen eine Messung der Absorption beziehungsweise Reflexion durchgeführt.

Derartige Messungen werden regelmäßig im Rahmen der Wareneingangsprüfung und während des Produktionsprozesses durchgeführt. Aufgrund der strengen Hygiene-Vorschriften ist es dabei notwendig, eine Verkeimung des Spektrometers sicher zu verhindern.

Als besonders problematisch haben sich dabei Antriebe für den Probenhalter herausgestellt, mit deren Hilfe der Probenhalter bewegt wird, um an verschiedenen Stellen der Probe Messungen durchzuführen. Die Antriebe weisen regelmäßig Hinterschneidungen auf, in denen sich Reste des Lebensmittels sammeln und dort Keimnester bilden können.

Aus der WO 2005/093433 ist ein magnetgeriebenes Probentransportsystem bekannt. Dabei werden Bewegungen von Magneten durch eine nichtmagnetische Trennplatte hindurch auf Ferromagneten enthaltende Probenträger übertragen.

Aus US 2 498 030 A ist ein Probentisch zur Untersuchung einer Probe unter Verwendung von Licht bekannt, bei dem ein Probenhalter beweglich auf einem Transportband angeordnet ist, das sich teilweise in einem Gehäuse und teilweise außerhalb des Gehäuses erstreckt. In US 7 105 338 B1 wird ein Drehtisch für Petri-Schalen beschrieben, der unter Anwendung magnetischer Kräfte angetrieben wird.

Bei bekannten Spektrometern wird außerdem der Lichtstrahl über die Probe geführt, um Keimnester zu verhindern. Dadurch wird ein Antrieb des Probenhalters überflüssig und Keimnester werden vermieden. Zum Führen des Lichtstrahls werden beispielsweise bewegliche Spiegel verwendet.

Die Steuerung dieser Spiegel ist jedoch aufwendig und insbesondere bei rauen Umgebungsbedingungen, wie beispielsweise in der Warenannahme, fehleranfällig.

Es ist daher die Aufgabe der vorliegenden Erfindung, einen Probentisch für eine Lebensmittelanalysevorrichtung vorzuschlagen, der die obigen Nachteile vermindert. Insbesondere ist es Aufgabe der Erfindung, einen vereinfachten Probentisch und eine Lebensmittelanalysevorrichtung anzugeben, die eine schnelle oder einfache spektroskopische Analyse einer Lebensmittelprobe ermöglichen und leicht zu reinigen sind.

Die Erfindung löst die Aufgabe durch einen Probentisch für eine Lebensmittelanalysevorrichtung bzw. eine Lebensmittelanalysevorrichtung mit den Merkmalen der Ansprüche 1 und 12.

Vorteilhaft an dem erfindungsgemäßen Probentisch ist, dass auf eine Führung des Lichtstrahls verzichtet werden kann. Das reduziert die Zahl der anzusteuernden Teile, wodurch der Probentisch beziehungsweise ein Lebensmittelanalysevorrichtung, die einen erfindungsgemäßen Probentisch umfasst, weniger fehleranfällig und kostengünstiger herzustellen ist.

Vorteilhaft ist zudem, dass der Probenhalter leicht abzunehmen und zu reinigen ist. So ist es beispielsweise möglich, in regelmäßigen Abständen den Probenhalter zu wechseln und zu reinigen. Zum Abnehmen des Probenhalters müssen dabei keine mechanischen Verbindungen gelöst werden. Es ist zudem möglich, den Probenhalter so auszubilden, dass er keine Hinterschneidungen aufweist. Dadurch werden Ansammlungen des Lebensmittels und damit Keimnester vermieden.

Ein weiterer Vorteil der vorliegenden Erfindung ist, dass eine große Zahl von Probenhaltern vorgehalten werden kann, die schnell mit dem übrigen Probentisch verbindbar sind, ohne mechanische Verbindungen lösen zu müssen.

Ein weiterer Vorteil ist, dass das Gehäuse des Probetischs wasserdicht kapselbar aufgeführt werden kann, so dass der Probentisch regelmäßig durch Abspritzen reinigbar ist. Eine derartige Form der Reinigung ist schnell und kostengünstig möglich und verhindert sicher eine Verkeimung des Probentischs.

Unter einem Probenhalter wird dabei insbesondere eine Vorrichtung verstanden, in die eine Probe eines Lebensmittels eingefüllt werden kann. Unter einem Probenhalter wird insbesondere aber auch eine Vorrichtung verstanden, in die eine weitere Vorrichtung einsetzbar ist, die wiederum die Probe des Lebensmittels enthält. Bei letzterer Vorrichtung kann es sich beispielsweise um eine Petrischale handeln.

Unter einem Bewegungsmittel wird insbesondere eine Vorrichtung verstanden, die den Probenhalter auf einer Kreisbahn bewegen kann. Das Bewegungsmittel ist insbesondere ausgebildet zum Drehen des Probenhalters um eine Drehachse. Handelt es sich bei dem Probenhalter um einen Probenhalter zur Aufnahme einer Petrischale, so verläuft die Drehachse vorzugsweise durch die Grundflächen der Petrischale, insbesondere durch deren Mittelpunkt.

Das Gehäuse ist gemäß der Erfindung so ausgebildet, dass es eine bei Betrieb des Probentischs nach oben weisende Oberseite aufweist, die im wesentlichen plan ist. Der Probenhalter ist dann zum Gleiten auf dieser planen Oberfläche ausgebildet. Dazu koppelt der Probenhalter erfindungsgemäß magnetisch an das Bewegungsmittel an.

Unter einer magnetischen Ankopplung ist dabei zu verstehen, dass eine Bewegung des Bewegungsmittels auf Grund magnetischer Anziehungs- beziehungsweise Abstoßungskräfte zu einer Bewegung des Probenhalters führt. Besonders vorteilhaft ist, das Gehäuse hinterschneidungsfrei auszubilden, so dass sich keine Keimnester bilden können.

In einer bevorzugten Ausführungsform umfasst das Bewegungsmittel mindestens einen Antriebsmagneten und der Probenhalter mindestens einen Mitnehmermagneten.

Diese sind vorzugsweise so gekoppelt, dass sich unterschiedliche Polarität (Nord- beziehungsweise Südpol) von Mitnehmermagneten bzw. Antriebsmagneten auf beiden Seiten des Gehäuses gegenüberliegt. Liegt beispielsweise eine Nordpolseite des Antriebsmagneten am Gehäuse an, so liegt auf der gegenüberliegenden Seite eine Südpolseite des Mitnehmermagneten. Die magnetischen Feldlinien durchsetzen dann das Gehäuse, so dass dann, wenn der Antriebsmagnet vom Bewegungsmittel bewegt wird, der Mitnehmermagnet eine magnetische Kraft erfährt, wodurch dieser der Bewegungsantriebsmagneten folgt.

Bevorzugt ist das Bewegungsmittel ausgebildet, um den Probenhalten auf einer Kreisbahn zu bewegen und/oder um seine horizontale Achse zu drehen. Alternativ oder auch additiv ist ein x-y-Tisch vorgesehen, um den Probenhalter frei im Raum positionieren zu können. Die x-y-Ebene verläuft dabei parallel zu dem Gehäuseteil, auf dem der Probenhalter bei Betrieb aufliegt. Das ist bevorzugt eine horizontale Oberseite des Gehäuses.

Bevorzugt weist der Probentisch ein Fenster auf zum Durchtritt von Licht, wobei das Fenster so angeordnet ist, dass ein senkrecht aus dem Fenster austretender Lichtstrahl auf die im Probenhalter aufgenommene Probe des Lebensmittels trifft. Je nach für die Messung vorgesehenem Wellenlängenspektrum ist das Fenster für die entsprechenden Wellenlängen im Wesentlichen durchlässig.

Vorzugsweise ist der Probenhalter zur Aufnahme einer Petrischale ausgebildet. Insbesondere ist der Probenhalter ausgebildet zur Aufnahme von Petrischalen mit einem Durchmesser von mehr als 30 mm, insbesondere von mehr als 50 mm, insbesondere von mehr als 70 mm, insbesondere von mehr als 90 mm. Insbesondere ist der Probenhalter ausgebildet zur Aufnahme von Petrischalen mit einem Durchmesser von weniger als 300 mm, insbesondere von weniger als 200 mm, insbesondere von weniger als 150 mm, insbesondere von weniger als 120 mm.

Bevorzugt ist das Gehäuse wasserdicht ausgebildet. Das heißt, dass es insbesondere mit einem Wasserstrahl mit Leitungsdruck von 4 MPa abspritzbar ist, ohne dass Wasser in das Gehäuse eindringt. Das Gehäuse wird zudem dann als wasserdicht betrachtet, wenn es so unter Wasser platziert werden kann, dass es gerade vollständig von Wasser bedeckt ist und dennoch kein Wasser in das Gehäuse eindringt.

Vorzugsweise ist das Gehäuse aus V2A- oder V4A-Stahl gefertigt. Alternativ ist das Gehäuse aus Aluminium oder lackiertem Kunststoff gefertigt. Dabei ist das Gehäuse vorzugsweise unmagnetisch. Bevorzugt werden im Rahmen der Erfindung Gehäusematerialien, die desinfizierbar sind, insbesondere desinfizierbarer Stahl. Die Oberfläche des Stahls ist so ausgebildet, dass sie einfach zu desinfizieren ist.

Vorteilhafterweise ist der Probentisch von außen ein- und ausschaltbar, wozu beispielsweise ein Schalter innerhalb des Gehäuses angeordnet ist, der von außen ohne mechanische Veränderung der Oberfläche des Gehäuses steuerbar ist. Ein solcher Schalter kann ein durch einen von außen gehaltenen Magneten beeinflusster Reed-Kontakt sein. Es ist jedoch auch möglich, andere Schalter zu verwenden, beispielsweise solche, die auf eine Berührung des Gehäuses an einer bestimmten Stelle reagieren.

Der Probentisch ist vorzugsweise drahtlos fernsteuerbar und auslesbar. Dies bietet den Vorteil, dass eine Datenübertragung von und zu dem Probentische möglich ist, ohne dass dessen Eignung zur Desinfektion beeinträchtigt wird. Der Probentisch umfasst vorzugsweise einen Anzeigeschirm (Display), besonders bevorzugt einen Touch-Screen, wobei der Anzeigeschirm zum Durchtritt elektromagnetischer Strahlung geeignet ist, und umfasst einen innerhalb des Gehäuses angeordneten Übertrager zum Übertragen elektromagnetischer Signale, wobei der Übertrager relativ zu dem Anzeigeschirm, insbesondere dem Touch-Screen so angeordnet ist, dass eine Übertragung von elektromagnetischen Signalen von und zu dem Übertrager durch den Anzeigeschirm möglich ist. Alternativ sind auch andere Möglichkeiten anwendbar, um eine drahtlose Übertragung von Signalen zu ermöglichen, beispielsweise eine Infrarot-Schnittstelle. Auch kann der Anzeigeschirm durch eine andere geeignete elektromagnetische Öffnung des Gehäuses, beispielsweise durch ein Glasfenster, ersetzt werden. Der Anzeigeschirm bietet jedoch den Vorteil, dass seine Oberfläche desinfizierbar ausgeführt sein kann, so dass eine einfache Bedienung des Probentisches ohne Einschränkung der Desinfizierbarkeit des Gehäuses möglich ist. Als Übertrager können z. B. Bluetooth-, WLAN- oder andere geeignete Schnittstellen verwendet werden.

Bevorzugt erfolgt die Ableitung von im Betrieb im Gehäuse entstehender Wärme durch das Gehäuse. Dazu können im Gehäuse Wärmeableitmittel angeordnet und mit dem Gehäuse verbunden werden, beispielsweise Aluminiumbrücken oder Wärmeleitpaste, so dass das Gehäuse als Kühlkörper funktioniert. Die Wärmeableitmittel werden zwischen Wärme erzeugenden Einbauten, beispielsweise einem Transformator, und dem Gehäuse angeordnet. Das Wärmeableitmittel kann auch ein Befestigungsrahmen sein, der einen Motor oder ähnliches trägt und am Gehäuse befestigt ist. Dies macht Kühlungsschlitze überflüssig.

Im Folgenden wird die Erfindung anhand der beigefügten Zeichnungen näher erläutert. Dabei zeigen:
- Figur 1: eine perspektivische Ansicht einer erfindungsgemäßen Lebensmittelanalysevorrichtung mit einem erfindungsgemäßen Probentisch, in dessen Probenhalter eine Probe eines Lebensmittels aufgenommen ist,
- Figur 2: eine Lebensmittelanalysevorrichtung gemäß Figur 1, jedoch ohne Probe eines Lebensmittels,
- Figur 3: die Lebensmittelanalysevorrichtung nach Figur 1, die zusätzlich eine Abdeckung aufweist,
- Figur 4: eine Innenansicht einer erfindungsgemäßen Lebensmittelanalysevorrichtung, und
- Figur 5: eine schematische Ansicht eines erfindungemäßen Probenhalters einer Lebensmittelanalysevorrichtung.

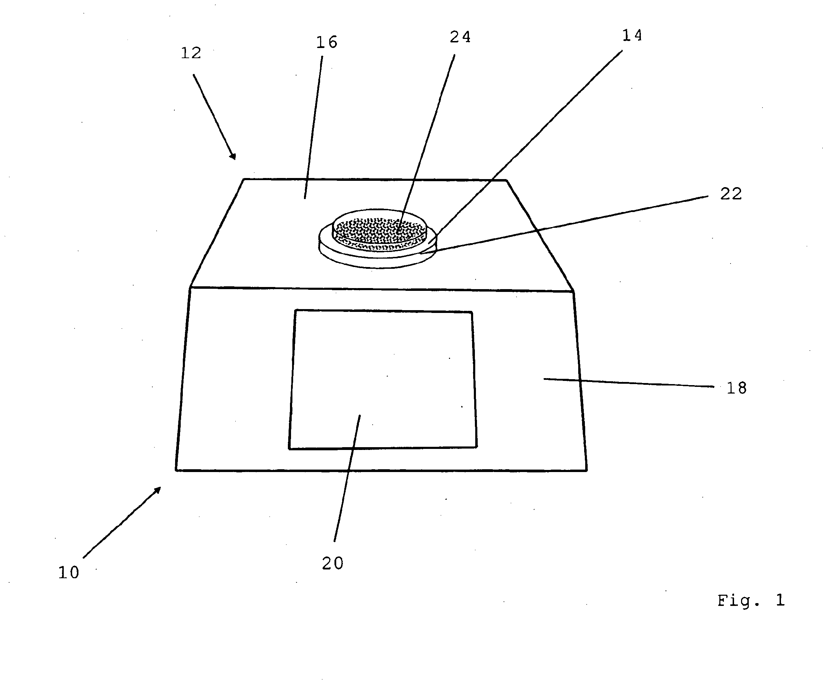

Figur 1 zeigt einen Probentisch 10, der ein Gehäuse 12 und einen Probenhalter 14 aufweist. Das Gehäuse 12 hat eine Oberseite 16, die im Wesentlichen horizontal verläuft, und eine Frontseite 18, die unter einem Winkel von ungefähr 45° gegen die Horizontale geneigt ist. Das Gehäuse 12 besteht aus V2A-Stahl. In einer alternativen Ausführungsform steht das Gehäuse aus V4A-Stahl. Die Oberfläche des Gehäuses 12 ist derart behandelt, dass sie desinfizierbar ist, so dass das Gehäuse 12 für die Lebensmittelanalyse geeignet ist. In der Frontseite 18 ist ein Display 20 eingelassen zur Anzeige von Messergebissen und zur Anzeige von Bedienungsmenüs. Das Display 20 ist vorzugsweise für elektromagnetische Strahlung durchlässig. Ein Touch-Screen bietet den Vorteil, dass eine Bedienung der Menüs mit dem Touch-Screen möglich ist. Das Gehäuse 12 ist allseits geschlossen und frei von Lüftungsschlitzen.

Der Probenhalter 14 umfast einen Kunststoffring 22, dessen Innendurchmesser so ausgebildet ist, dass eine Petrischale 24 mit 100 mm Durchmesser aufnehmbar ist. Der Probenhalter 14 ist ausgebildet, um auf der Oberseite 16 des Gehäuses 12 reibungsarm zu gleiten.

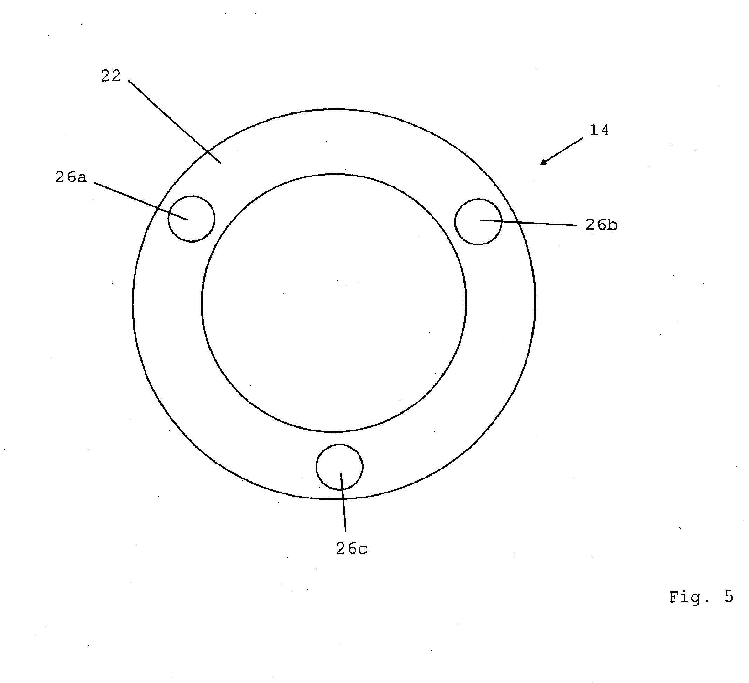

In den Kunststoffring 22 des Probenhalters 14 sind drei Mitnehmermagneten 26a, 26b, 26c (in Figur 1 nicht eingezeichnet)eingelassen (Figur 5). Die Magneten 26a, 26b, 26c schließen bündig mit der der Oberseite 16 des Gehäuses 12 zugewandten Unterseite des Kunststoffrings 22 ab. Die Nordpole der Mitnehmermagneten 26a, 26b, 26c weisen im Betrieb des Probentischs 10 alle nach oben.

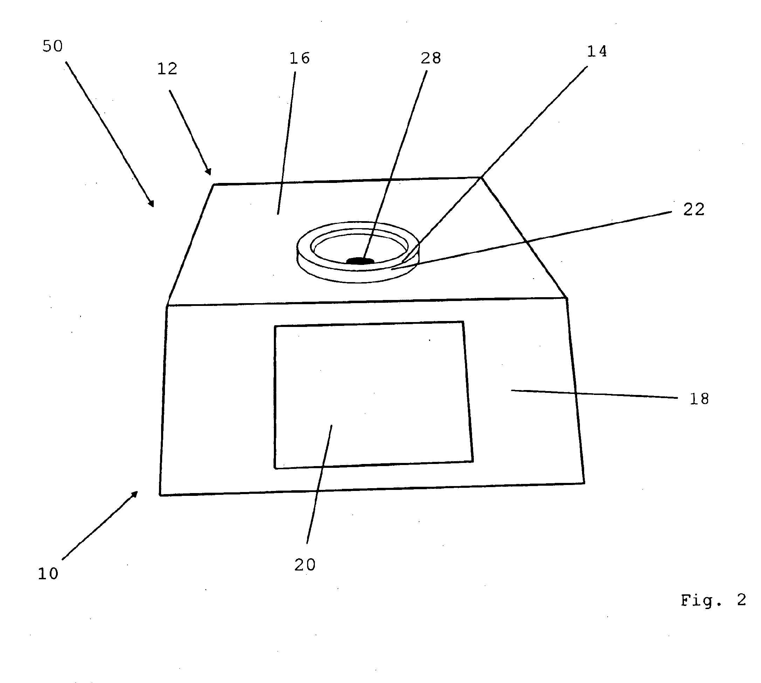

Figur 2 zeigt den Probentisch 10 mit dem Probenhalter 14 der jedoch keine Petrischale in sich aufgenommen hat. In der O-berseite 16 des Gehäuses 12 ist ein Fenster 28 eingelassen. Dieses Fenster ist eine Öffnung in der Oberseite 16 des Gehäuses 12, die mit einer durchsichtigen Scheibe abgedeckt ist. Das Fenster ist ausgebildet, um Licht im Spektralbereich von 380 bis 2500 nm, bevorzugt im Spektralbereich von 730 nm bis 1100 nm weitgehend absorptionsfrei passieren zu lassen.

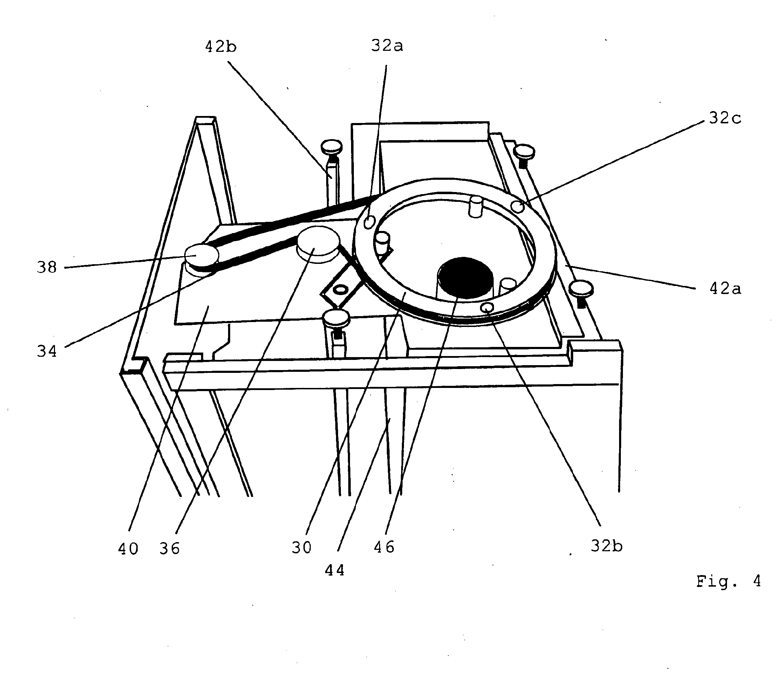

Figur 4 zeigt den Probentisch 10, wobei vom Gehäuse 12 die Oberseite 16 und die Frontseite 18 entfernt worden sind. Unmittelbar unterhalb der (hier nicht vorhandenen) Oberseite 16 des Gehäuses 12 befindet sich ein Antriebsring 30, der den gleichen Durchmesser aufweist wie der Kunststoffring 22 (vergleiche Figur 1).

In den Antriebsring 30 sind drei Antriebsmagneten 32a, 32b, 32c eingelassen, die bündig mit der Oberseite des Antriebsrings 30 abschließen. Die Nordpole der Antriebsmagneten 32a, 32b, 32c weisen alle nach oben und wirken im Betrieb mit den nach unten weisenden Südpolen der Mitnehmermagneten 26a, 26b, 26c zusammen. Der Antriebsring 30 ist unmittelbar unterhalb der Oberseite 16 des Gehäuses 12 angeordnet.

Der Antriebsring 30 wird über einen Riemen 34 in Form eines Zahnriemens angetrieben, der wiederum von einem Elektromotor 36 angetrieben ist. Die Bewegung des Riemens 34 wird über einen Winkelmesser 38 aufgenommen, der mit einer hier nicht eingezeichneten zentralen Steuerung verbunden ist und die die Drehung des Antriebsrings steuert.

Der Antriebsring 30 und der Elektromotor 36 sind auf einem Schlitten 40 angeordnet, der über zwei Führungsschienen 42a, 42b translatorisch verschiebbar ist.

Im Betrieb des Probentischs 10 dreht der Elektromotor 36 über den Riemen 34 den Antriebsriemen 30, so dass die Antriebsmagneten 32a, 32b, 32c direkt unterhalb der Oberseite 16 des Gehäuses 12 rotieren. Dadurch erfahren die Mitnehmermagneten 26a, 26b, 26c, die auf der gegenüber liegenden Seite der Oberseite 16 des Gehäuses 12 angeordnet sind, eine magnetische Kraft, so dass der Probenhalter 14 der Bewegung des Antriebsrings 30 folgt.

In der vorliegenden Ausführungsform sind sowohl die Antriebsmagneten 32a, 32b, 32c als auch die Mitnehmermagneten 26a, 26b, 26c äquidistant über den Umfang des Antriebsrings 30 beziehungsweise des Kunststoffrings 22 verteilt. Alternativ können auch 2, 4, 5, 6, 7, 8, 9, 10 oder mehr als 10 Antriebs- bzw. Mitnehmermagneten vorgesehen sein.

Die Antriebs- und Mitnehmermagneten sind dabei so stark gewählt, dass eine sichere Bewegung des Kunststoffrings 22 gewährleistet ist, wenn der Abstand zwischen den Antriebsmagneten 32a, 32b, 32c und den Mitnehmermagneten 26a, 26b, 26c 4 mm beträgt. Es handelt sich bei den Mitnehmer- beziehungsweise Antriebsmagneten um Permanentmagneten aus seltenen Erden. Als geeignet haben sich Magneten aus Kobalt-Samarium- oder Neodym-Eisen-Bor-Verbindungen bzw. -Legierungen herausgestellt. Es ist jedoch nicht zwingend notwendig, sowohl Antriebsmagneten, als auch Mitnehmermagneten vorzusehen. Sofern die Antriebsmagneten bzw. die Mitnehmermagneten stark genug ausgebildet sind, kann auf Mitnehmermagneten bzw. Antriebsmagneten verzichtet werden. Statt dessen sind dann Blöcke aus ferromagnetischem Material vorgesehen.

Figur 4 zeigt neben dem Probentisch 10 eine Lichtquelle 44 zum Erzeugen von Licht verschiedener Wellenlängen. Die Lichtquelle 44 umfasst beispielsweise eine Weißlichtquelle und einen Monochromator, wie es von herkömmlichen Spektralfotometern bekannt ist. Das Licht verlässt die Lichtquelle 44 durch eine Austrittsöffnung 46. Diese Austrittsöffnung 46 ist so angeordnet, dass ein aus der Austrittsöffnung 46 austretender Lichtstrahl durch das Fenster 28 in der Oberseite 16 des Gehäuses 12 tritt.

Die Austrittsöffnung 46 ist dabei so relativ zum Antriebsring 30 angeordnet, dass auch bei Drehung des Antriebsrings 30 die Austrittöffnung 46 niemals durch den Antrittsring 30 verdeckt ist. Entsprechend ist der Probenhalter 14 so ausgebildet, dass ein aus dem Fenster 28 austretender Lichtstrahl nicht vom Kunststoffring 22 verdeckt wird. Auch bei Drehung von Antriebsring 30 und Kunststoffring 22 kann somit stets Licht durch die Austriebsöffnung 46 und das Fenster 28 austreten und auf die Petrischale 24 treffen.

Durch den Schlitten 40 ist der Antriebsring 30 so verschiebbar, dass Licht durch die Austrittsöffnung 46 austreten kann, ohne auf eine im Probenhalter 14 aufgenommene Probe zu treffen. Auf diese Weise können Referenz- und Kalibriermessungen durchgeführt werden.

Trifft Licht wie beschrieben auf ein Lebensmittel in der Petrischale 24, so reflektiert dieses das Licht mit einer von der Wellenlänge abhängigen Rate. Ein Teil des so reflektierten Lichts gelangt durch das Fenster 28 zurück in das Gehäuse 12 und durch die Austrittsöffnung 46 in eine hier nicht eingezeichnete Analysevorrichtung 48.

Die Analyseeinrichtung 48 erfasst die von der Probe des Lebensmittels reflektierte Strahlung und leitet diese an eine ebenfalls nicht eingezeichnete Steuerung weiter. Die Steuerung umfasst eine Auswerteeinheit zum Auswerten der Messdaten.

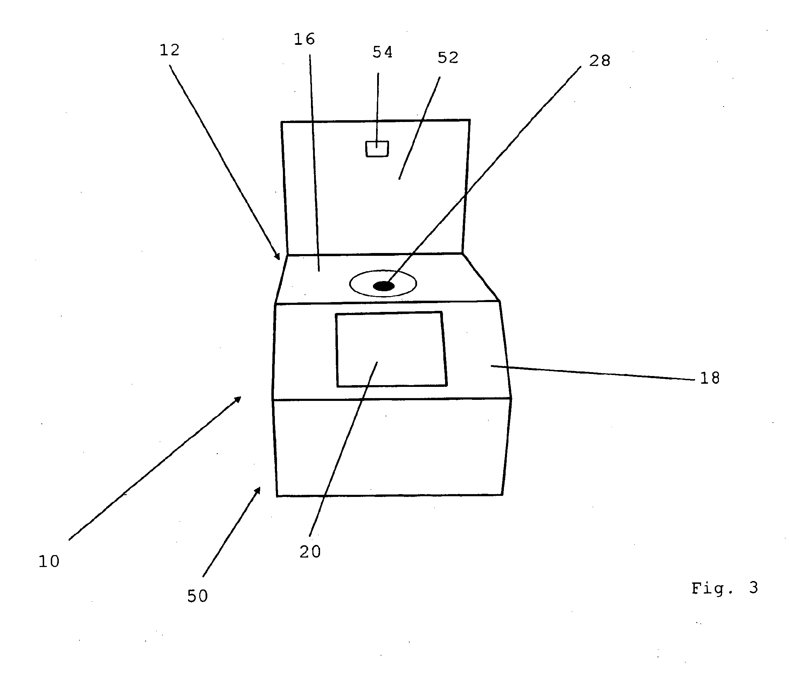

Figur 3 zeigt eine alternative Ausführungsform einer Lebensmittelanalysevorrichtung 50, die einen Probentisch 10 und eine Lichtquelle 44 wie oben beschrieben aufweist. Zusätzlich umfasst die Lebensmittelanalysevorrichtung 50 eine Abdeckung 52 in die ein Detektor 54 eingelassen ist. Dieser Detektor 54 detektiert Licht, das durch die Probe des Lebensmittels hindurch getreten ist, in Abhängigkeit von der Wellenlänge des Lichts.

Die vom Detektor 54 aufgenommene Messsignale werden über hier nicht eingezeichnete elektronische Kontakte oder durch eine Funkschnittstelle an die hier ebenfalls nicht eingezeichnete zentrale Steuerung der Lebensmittelanalysevorrichtung 50 weitergeleitet. Alternativ zum Detektor 54 umfasst die Abdeckung 52 einen Doppelspiegel 56 oder ein Prisma zum Umlenken des Lichtstrahls in das Gehäuse 12 zurück. In diesem Fall ist im Gehäuse 12 ein Detektor zum Detektieren des durch die Probe des Lebensmittels getretene Lichtmenge vorgesehen.

Es ist möglich, die Lebensmittelanalysevorrichtung 50 so auszubilden, dass sowohl das von der Probe des Lebensmittels reflektierte Licht, als auch das transmittierte Licht gemessen werden können.

Ein Verfahren zur Analyse einer Probe eines Lebensmittels wird ausgeführt, indem die Probe des Lebensmittels in den Strahlengang der Lebensmittelanalysevorrichtung gebracht wird, eine Absorptions- und/oder Transmissionsmessung durchgeführt wird und anschließend die Probe um einen vorher festgelegten Betrag bewegt wird. Diese Bewegung wird mit der erfindungsgemäßen Vorrichtung durchgeführt.

## Patentansprüche

1. Probentisch für eine Lebensmittelanalysevorrichtung zur Analyse einer Probe eines Lebensmittels mit
- einem Probenhalter (14) zur Aufnahme der Probe des Lebensmittels,
- einem Bewegungsmittel (36, 34, 30, 32, 26) zum Bewegen des Probenhalters (14), und
- einem das Bewegungsmittel umgebenden Gehäuse (12), wobei
- der Probentisch ein Fenster (28) zum Durchtritt von Licht aufweist, wobei das Fenster (28) im Gehäuse (12) so angeordnet ist, dass ein aus dem Fenster austretender Lichtstrahl auf die im Probenhalter (14) aufgenommene Probe des Lebensmittels trifft,
**dadurch gekennzeichnet, dass**
- das Bewegungsmittel (36, 34, 30, 32, 26) und der Probenhalter (14) magnetisch gekoppelt sind, so dass der Probenhalter (14) außerhalb des Gehäuses (12) relativ zum Gehäuse (12) bewegbar ist, wobei der Probenhalter (14) so ausgebildet ist, dass er auf der nach oben weisenden Oberseite des Gehäuses (12) gleitet, und
- das Gehäuse (12) allseits geschlossen ist.

2. Probentisch nach Anspruch 1, **dadurch gekennzeichnet, dass** das Bewegungsmittel (36, 34, 30, 32, 26) mindestens einen Antriebsmagneten (32) umfasst, und
der Probenhalter (14) mindestens einen Mitnehmermagneten (26) zum Zusammenwirken mit dem mindestens einen Antriebsmagneten (32) umfasst,
so dass der Probenhalter (14) durch das Zusammenwirken der Magneten (26, 32) relativ zum Gehäuse (12) bewegbar ist.

3. Probentisch nach einem der vorstehenden Ansprüche, **dadurch gekennzeichnet, dass** das Bewegungsmittel (36, 34, 30, 32, 26) einen Elektromotor und einen mit dem Elektromotor (36) gekoppelten Antriebsring (30) umfasst.

4. Probentisch nach einem der vorstehenden Ansprüche, **dadurch gekennzeichnet, dass** das Bewegungsmittel (36, 34, 30, 32, 26) eine Verschiebeeinheit (40) umfasst zum translatorischen Verschieben des Probenhalters (14) relativ zum Gehäuse (12).

5. Probentisch nach einem der vorstehenden Ansprüche, **dadurch gekennzeichnet, dass** der Antriebsring (30) drei Hochleistungsmagneten (32a, 32b, 32c) umfasst.

6. Probentisch nach einem der vorstehenden Ansprüche, wobei das Fenster (28) im Gehäuse (12) so angeordnet ist, dass ein senkrecht aus dem Fenster austretender Lichtstrahl auf die im Probenhalter (14) aufgenommene Probe des Lebensmittels trifft.

7. Probentisch nach einem der vorstehenden Ansprüche, **dadurch gekennzeichnet, dass** der Probenhalter (14) zur Aufnahme einer Petrischale (24) ausgebildet ist.

8. Probentisch nach einem der vorstehenden Ansprüche **dadurch gekennzeichnet, dass** das Gehäuse (12) wasserdicht ist.

9. Probentisch nach einem der vorstehenden Ansprüche, **dadurch gekennzeichnet, dass** das Gehäuse (12) aus nichtmagnetischem Material besteht, insbesondere aus V2A- oder V4A-Stahl.

10. Probentisch nach einem der vorstehenden Ansprüche **gekennzeichnet durch** einen Anzeigeschirm, der zum Durchtritt elektromagnetischer Strahlung geeignet ist, und einen innerhalb des Gehäuses angeordneten Übertrager zum Übertragen elektromagnetischer Signale, wobei der Übertrager relativ zu dem Anzeigeschirm so angeordnet ist, dass eine Übertragung von elektromagnetischen Signalen von und zu dem Übertrager **durch** den Touch-Screen möglich ist.

11. Probentisch nach Anspruch 10, **dadurch gekennzeichnet, dass** der Anzeigeschirm einen Touch-Screen umfasst.

12. Lebensmittelanalysevorrichtung zur spektroskopischen Untersuchung von Proben von Lebensmitteln mit
- einem Probentisch (10) nach einem der vorstehenden Ansprüche, der eine vom Gehäuse (12) umschlossene Lichtquelle (44) umfasst,
wobei der Probenhalter (14) so zum Fenster (28) angeordnet ist, dass bei Betrieb der Lebensmittelanalysevorrichtung (50) Licht der Lichtquelle (44) die im Probenhalter (14) aufgenommene Probe des Lebensmittels trifft, und
- einem Mittel (54) zum Analysieren einer physikalischen Größe, die für die Wechselwirkung der Probe mit Licht der Lichtquelle (44) charakteristisch ist, insbesondere der wellenlängenabhängigen Absorption des Lichts der Lichtquelle (44) durch die Probe des Lebensmittels.

13. Lebensmittelanalysevorrichtung nach Anspruch 12, **dadurch gekennzeichnet, dass** das Mittel (54) zum Analysieren der wellenlängenabhängigen Absorption im Gehäuse (12) angeordnet ist.

14. Lebensmittelanalysevorrichtung nach einem der Ansprüche 12 oder 13, **gekennzeichnet durch** einen Spiegel zum Reflektieren von Licht der Lichtquelle (44), das **durch** die Probe getreten ist, in das Gehäuse (12) zurück.

## Claims

1. A sample table for a food analyzing device for analyzing a sample of food, with
- a sample holder (14) for holding the sample of food,
- a movement means (36, 34, 30, 32, 26) for moving the sample holder (14), and
- a housing which surrounds the movement means, wherein
- the sample table has a window (28) for the passage of light, wherein the window (28) is arranged in the housing (12) such that a light beam emerging from the window impinges on the sample of food held in the sample holder (14),
**characterized in that**
- the movement means (36, 34, 30, 32, 26) and the sample holder (14) are magnetically coupled so that the sample holder (14) outside the housing (12) can be moved relative to the housing (12), wherein the sample holder (14) is formed such that it slides on the upward upper side of the housing (12), and
- the housing (12) is closed on all sides.

2. The sample table according to claim 1, **characterized in that**
the movement means (36, 34, 30, 32, 26) comprises at least one drive magnet (32), and
the sample holder (14) comprises at least one follower magnet (26) for interacting with the at least one drive magnet (32),
so that the sample holder (14) can be moved relative to the housing (12) as a result of the interaction of the magnets (26, 32).

3. The sample table according to one of the preceding claims, **characterized in that** the movement means (36, 34, 30, 32, 26) comprises an electric motor and a drive ring (30) coupled to the electric motor (36).

4. The sample table according to one of the preceding claims, **characterized in that** the movement means (36, 34, 30, 32, 26) comprises a displacement unit (40) for displacing the sample holder (14) in translation relative to the housing (12).

5. The sample table according to one of the preceding claims, **characterized in that** the drive ring (30) comprises three high-power magnets (32a, 32b, 32c).

6. The sample table according to one of the preceding claims, wherein the window (28) is arranged in the housing (12) in such a way that a light beam vertically emerging from the window impinges on the sample of food held in the sample holder (14).

7. The sample table according to one of the preceding claims, **characterized in that** the sample holder (14) is designed to hold a Petri dish (24).

8. The sample table according to one of the preceding claims, **characterized in that** the housing (12) is watertight.

9. The sample table according to one of the preceding claims, **characterized in that** the housing (12) is made from nonmagnetic material, in particular from V2A or V4A steel.

10. The sample table according to one of the preceding claims, **characterized by** a display screen, which is suitable for the passage of electromagnetic radiation, and a transmitter arranged inside the housing for transmitting electromagnetic signals, wherein the transmitter is arranged relative to the display screen in such a way that a transmission of electromagnetic signals from and to the transmitter through the display screen is possible.

11. The sample table according to claim 10, **characterized in that** the display screen comprises a touchscreen.

12. A food analyzing device for the spectroscopic analysis of samples of food, comprising:
- a sample table (10) according to one of the preceding claims, which comprises a light source (44) enclosed by the housing (12),
wherein the sample holder (14) is arranged with respect to the window (28) in such a way that, during operation of the food analyzing device (50), light from the light source (44) impinges on the sample of food held in the sample holder (14), and
- means (54) for analyzing a physical parameter which is characteristic of the interaction of the sample with light from the light source (44), in particular the wavelength-dependent absorption of the light from the light source (44) by the sample of food.

13. The food analyzing device according to claim 12, **characterized in that** the means (54) for analyzing the wavelength-dependent absorption is arranged in the housing (12).

14. The food analyzing device according to one of claims 12 or 13, **characterized by** a mirror for reflecting light from the light source (44), which has passed through the sample, back into the housing (12).

## Revendications

1. Table d'échantillonnage pour un dispositif d'analyse de produit alimentaire servant à l'analyse d'un échantillon d'un produit alimentaire, comprenant :
- un support d'échantillon (14) servant la réception de l'échantillon du produit alimentaire,
- un moyen de déplacement (36, 34, 30, 32, 26) servant au déplacement du support d'échantillon (14) et
- un boîtier (12) entourant le moyen de déplacement,
la table d'échantillonnage comportant une fenêtre (28) servant au passage de lumière, la fenêtre (28) étant disposée dans le boîtier (12) de sorte qu'un rayon lumineux sortant de la fenêtre soit incident sur l'échantillon du produit alimentaire reçu dans le support d'échantillon (14),
**caractérisée**
- **en ce que** le moyen de déplacement (36, 34, 30, 32, 26) et le support d'échantillon (14) sont couplés magnétiquement de sorte que le support d'échantillon (14) puisse être déplacé relativement au boîtier (12) en dehors du boîtier (12), le support d'échantillon (14) étant conçu de sorte qu'il glisse sur la surface supérieure du boîtier (12) tournée vers le haut, et
- **en ce que** le boîtier (12) est fermé de tous les côtés.

2. Table d'échantillonnage selon la revendication 1, **caractérisée en ce que** le moyen de déplacement (36, 34, 30, 32, 26) comporte au moins un aimant de commande (32), et
**en ce que** le support d'échantillon (14) comporte au moins un aimant entraîneur (26) servant à l'action conjointe avec ledit au moins un aimant de commande (32)
de sorte que le support d'échantillon (14) puisse être déplacé relativement au boîtier (12) par l'action conjointe des aimants (26, 32).

3. Table d'échantillonnage selon l'une quelconque des revendications précédentes, **caractérisée en ce que** le moyen de déplacement (36, 34, 30, 32, 26) comporte un moteur électrique et un anneau de commande (30) couplé au moteur électrique (36).

4. Table d'échantillonnage selon l'une quelconque des revendications précédentes, **caractérisée en ce que** le moyen de déplacement (36, 34, 30, 32, 26) comporte une unité de déplacement (40) servant au déplacement translatoire du support d'échantillon (14) relativement au boîtier (12).

5. Table d'échantillonnage selon l'une quelconque des revendications précédentes, **caractérisée en ce que** l'anneau de commande (30) comporte trois aimants de forte puissance (32a, 32b, 32c).

6. Table d'échantillonnage selon l'une quelconque des revendications précédentes, la fenêtre (28) étant disposée dans le boîtier (12) de sorte qu'un rayon lumineux sortant verticalement de la fenêtre soit incident sur l'échantillon du produit alimentaire reçu dans le support d'échantillon (14).

7. Table d'échantillonnage selon l'une quelconque des revendications précédentes, **caractérisée en ce que** le support d'échantillon (14) est conçu pour la réception d'une boîte de Pétri (24).

8. Table d'échantillonnage selon l'une quelconque des revendications précédentes **caractérisée en ce que** le boîtier (12) est étanche à l'eau.

9. Table d'échantillonnage selon l'une quelconque des revendications précédentes, **caractérisée en ce que** le boîtier (12) est composé d'un matériau non magnétique, en particulier de l'acier V2A ou V4A.

10. Table d'échantillonnage selon l'une quelconque des revendications précédentes **caractérisée par** un écran d'affichage, qui est approprié à la traversée d'un rayonnement électromagnétique et par un transmetteur, disposé à l'intérieur du boîtier, servant à la transmission de signaux électromagnétiques, le transmetteur étant disposé relativement à l'écran d'affichage de sorte qu'une transmission de signaux électromagnétiques soit possible par le biais de l'écran tactile depuis ou vers le transmetteur.

11. Table d'échantillonnage selon la revendication 10, **caractérisée en ce que** l'écran d'affichage comporte un écran tactile.

12. Dispositif d'analyse de produit alimentaire servant à l'examen spectroscopique d'échantillons de produit alimentaire, comprenant :
- une table d'échantillonnage (10), selon l'une quelconque des revendications précédentes, qui comporte une source lumineuse (44) confinée par le boîtier (12),
le support d'échantillon (14) étant disposé vers la fenêtre (28) de sorte que, en cas d'utilisation du dispositif d'analyse de produit alimentaire (50), la lumière de la source lumineuse (44) soit incidente sur l'échantillon du produit alimentaire reçu dans le support d'échantillon (14), et
- un moyen (54) servant à l'analyse d'une grandeur physique qui est caractéristique de l'interaction de l'échantillon avec la lumière de la source lumineuse (44), en particulier de l'absorption dépendante de la longueur d'onde de la lumière de la source lumineuse (44) par l'échantillon du produit alimentaire.

13. Dispositif d'analyse de produit alimentaire selon la revendication 12, **caractérisé en ce que** le moyen (54) servant à l'analyse de l'absorption dépendante de la longueur d'onde est disposé dans le boîtier (12).

14. Dispositif d'analyse de produit alimentaire selon l'une quelconque des revendications précédentes 12 ou 13, **caractérisé par** un miroir servant à renvoyer dans le boîtier (12) par réflexion la lumière de la source lumineuse (44) qui est incidente sur l'échantillon.
